# FASCICULE DE BREVET EUROPEEN

(11) **EP 1 088 525 B1**
(45) Date de publication et mention de la délivrance du brevet: **19.09.2007**
(21) Numéro de dépôt: 00410120.0
(22) Date de dépôt: 28.09.2000
(51) Int. Cl.: A61B 19/00

(54) **Procédé de recalage d'images médicales sur un patient et dispositif associé**
Verfahren zur Ortungszurückstellung von zwei medizinischen Bildern an einem Patienten und Vorrichtung dafür
Registration process of two medical images from a patient and its device

(30) Priorité: 01.10.1999 FR 9912525
(43) Date de publication de la demande: 04.04.2001
(73) Titulaire: Perception Raisonnement Action En Medecine, 38700 La Tronche (FR)
(72) Inventeur: Lavallée, Stéphane, 38660 Saint Vincent de Mercuze (FR); Schmerber, Sébastien, 38000 Grenoble (FR)
(74) Mandataire: de Beaumont, Michel

(56) Documents cités:
- US-A- 4 465 069
- US-A- 4 617 925
- US-A- 5 474 564
- US-A- 5 807 252

## Description

La présente invention concerne le domaine de l'imagerie médicale applicable à la chirurgie assistée par ordinateur.

Un appareillage classique utilisé pour la chirurgie assistée par ordinateur, ou plus précisément les gestes médicaux chirurgicaux assistés par ordinateur, comporte un système de localisation tridimensionnel qui détermine les positions et orientations spatiales de moyens de repérage montés sur des instruments, des pointeurs, des capteurs ou des structures anatomiques, système de localisation qui peut être optique, magnétique, mécanique, à ultrasons, à inertie, etc.

Dans ce type d'intervention chirurgicale, on effectue, préalablement à l'intervention, des images médicales à trois dimensions du patient à opérer. Ces images peuvent être effectuées plusieurs jours avant l'intervention et elles sont réalisées dans la salle où se trouve l'appareillage d'imagerie médicale (scanner, résonance magnétique IRM, etc.). Lors de l'intervention chirurgicale, on veut pouvoir observer sur un écran simultanément les images préenregistrées et la position d'un outil d'intervention agissant sur le patient. Il faut donc transposer dans le référentiel opératoire les images préenregistrées. En d'autres termes, il faut recaler le référentiel des images préenregistrées sur un référentiel lié patient.

Pour cela, de façon classique, on fait correspondre des points particuliers du patient ou des structures matérielles avec les mêmes points ou structures repérés dans les images préenregistrées. En outre, une fois que cette mise en correspondance a été effectuée une fois, on veut pouvoir la retrouver si le patient bouge, s'il est déplacé ou lors d'une autre intervention. Pour cela, on fixe au patient un corps de référence dont les mouvements, suivis par le système de localisation, sont déterminés et utilisés pour recaler les images préenregistrées sur le patient.

Divers moyens de fixation de corps de référence sont possibles.

Le brevet américain N°5 474 564 décrit un dispositif formé de deux arcs et comportant un appui nasal et des appuis sur l'arcade sourcilière.

Le brevet américain N°4 617 925 décrit un adaptateur destiné à déterminer la position de structures du cerveau d'un patient et comportant plusieurs supports dont un s'adapte aux oreilles du patient.

En neurochirurgie et en radiothérapie du cerveau, des systèmes connus utilisent un anneau placé autour du crâne et sur lequel sont installés des marqueurs visibles par le système de localisation. Il s'agit en général de cadres dits stéréotaxiques relativement légers dont la fixation se fait par serrage autour de la tête ou par fixation dans la boîte crânienne.

En chirurgie ORL, des systèmes connus utilisent un casque souple qui englobe la tête du patient et que l'on serre globalement autour de la tête, ainsi que des systèmes utilisant un appui nasal et des plots qui se placent dans les oreilles. Ces deux types de systèmes n'offrent pas une bonne stabilité dans le temps et peuvent générer des imprécisions importantes, surtout s'ils sont démontés et remontés sur le patient.

En chirurgie orthopédique, il est connu de placer dans l'os opéré des tiges avec vis sur lesquelles sont ensuite fixés les moyens de repérage ; plus particulièrement, en chirurgie du rachis, on place en général une pince avec des mors que l'on vient serrer sur les apophyses épineuses des vertèbres opérées. Les moyens de repérage sont ensuite installés sur ces pinces. Ces fixations sont rigides et invasives.

Aucun de ces dispositifs de fixation n'amène le corps de référence à présenter une position prédéterminée avec la structure anatomique du patient et, pour recaler le référentiel des images tridimensionnelles préétablies sur le référentiel du moyen de repérage, il faut d'une part repérer la position du corps de référence par rapport au patient, et d'autre part recaler les structures anatomiques du patient sur le référentiel du corps de référence. Cela présente l'inconvénient de nécessiter le repérage de points anatomiques du patient et peut être source d'erreurs. En outre, si pour une raison quelconque on est amené à retirer du patient le dispositif de fixation, il sera nécessaire d'effectuer à nouveau ce repérage initial de points anatomiques du patient pour déterminer la nouvelle relation entre la position du nouvel emplacement du dispositif de fixation et la structure anatomique du patient.

Par ailleurs, on connaît des dispositifs de fixation qui épousent la forme des structures anatomiques.

Ainsi, en radiothérapie générale, on a utilisé des matelas anatomiques qui épousent la forme du corps du patient ; ces matelas, par exemple des matelas thermogonflables, outre la gêne qu'ils procurent, n'offrent en pratique qu'une précision médiocre.

On connaît aussi l'utilisation de moules ou gabarits sur mesure pour chaque patient tels qu'ils coïncident avec la forme externe d'une structure anatomique définie sur des images médicales de cette structure. Cette solution a l'inconvénient de nécessiter la réalisation d'un gabarit individualisé pour chaque patient, ce qui est lourd à mettre en oeuvre et augmente les coûts.

On a aussi utilisé des moulages dentaires se fixant sur la mâchoire ou la mandibule du patient ; des moyens de repérage sont fixés sur ces moulages dentaires et permettent le repérage de la position des moulages par le système de localisation. Là encore, cette solution présente l'inconvénient de nécessiter un moulage individualisé pour chaque patient. En outre, un inconvénient de ce système est que le moule dentaire doit être pressé sur les dents tout au long de l'utilisation du système et, par ailleurs, l'encombrement de ces moules peut être gênant pour certains types de chirurgie (maxillo-faciale par exemple) ou pour le placement de tuyaux de respiration.

Pour effectuer le recalage, l'art antérieur utilise trois procédés.

Ainsi, dans des procédés connus, le corps de référence est placé sur le patient au moment de l'examen d'imagerie. Ce corps de référence peut être un des dispositifs décrits ci-dessus, comme l'appui nasal comportant des plots placés dans les oreilles, ou des structures matérielles, comme des vis, boules, anneaux, collées sur la peau ou insérées dans un os du patient. Ce corps de référence est retrouvé facilement sur les images obtenues, par exemple par des logiciels. La position du corps de référence ne doit pas varier et ces procédés présentent l'inconvénient de devoir laisser, sur le patient, le corps de référence entre l'examen d'imagerie et l'intervention chirurgicale. En pratique, il est souvent nécessaire d'effectuer un nouvel examen d'imagerie juste avant l'intervention.

Dans d'autres procédés connus, le corps de référence n'est pas placé sur le patient pendant l'examen d'imagerie. Ces procédés utilisent un recalage à l'aide d'un certain nombre de points anatomiques particuliers du patient. Lors de l'intervention, un opérateur ou un chirurgien pointe des points particuliers du patient avec un capteur pour les repérer par rapport au système de localisation. Des logiciels retrouvent ces points particuliers sur les images, ce qui permet le recalage de l'image préenregistrée au cours de l'intervention. L'inconvénient majeur de ces procédés est que le repérage de points anatomiques présente nécessairement une certaine imprécision. Cela peut conduire à des erreurs importantes, notamment en rotation.

D'autres procédés plus précis utilisent des logiciels mettant en concordance des nuages de points appartenant à deux surfaces. Un nuage de points ( 20 points, par exemple) est repéré par le chirurgien sur une surface anatomique du patient et le recalage entre cette surface et la surface homologue repérée sur les images est effectué par des logiciels utilisant des algorithmes de convergence itératifs. Ces procédés présentent l'inconvénient de nécessiter des temps de calcul longs ou d'être peu fiables s'ils ne démarrent pas d'une position initiale proche de la solution.

Un objet de la présente invention est de prévoir un procédé pour recaler le référentiel d'une image tridimensionnelle d'un patient sur le référentiel d'un patient qui soit simple à mettre en oeuvre.

Un objet de la présente invention est de prévoir un procédé pour recaler le référentiel d'une image tridimensionnelle d'un patient sur le référentiel d'un patient qui soit fiable et qui autorise des déplacements du patient.

Un objet de la présente invention est prévoir un procédé ne nécessitant pas l'étape consistant à repérer, dans le référentiel opératoire, la position de plusieurs points anatomiques d'un patient.

Un objet de la présente invention est de prévoir un dispositif de fixation d'un corps de référence qui soit repérable par un système de localisation et qui soit facile à poser.

Un objet de la présente invention est de prévoir un dispositif qui puisse s'appliquer à une large série de patients.

Un objet de la présente invention est de prévoir un dispositif qui présente une bonne stabilité tout au long de l'intervention.

Un objet de la présente invention est de prévoir un dispositif qui présente une position sensiblement déterminée par rapport à la structure anatomique sur lequel il repose.

Pour atteindre ces objets, la présente invention prévoit un procédé pour recaler le référentiel d'un dispositif placé sur un patient au cours d'une intervention avec le référentiel d'une image tridimensionnelle préenregistrée de ce patient. Le procédé comprend les étapes suivantes
a) placer sur le patient un dispositif applicable à plusieurs patients et présentant une relation de position prédéterminée avec la structure anatomique du patient ;
b) repérer la position dudit dispositif par rapport à un système de localisation ; et
c) à l'aide du repérage dudit dispositif et de la relation de position prédéterminée du dispositif avec la structure anatomique du patient, déterminer une relation entre le référentiel du dispositif et le référentiel de ladite image.

Selon un mode de réalisation, l'étape consistant à déterminer la relation entre le référentiel du dispositif et le référentiel de ladite image comprend les étapes suivantes :
déterminer, par rapport audit dispositif, la position de points appartenant à la surface anatomique du patient,
utiliser des algorithmes de recalage itératifs pour déterminer dans l'image préenregistrée la position de cette surface anatomique, ces algorithmes utilisant la relation de position prédéterminée du dispositif avec la structure anatomique pour débuter leur recherche, et
déterminer une matrice de passage définissant la relation entre le référentiel du dispositif et le référentiel de ladite image.

Selon un mode de réalisation, dans le cas où le patient garde une position sensiblement stable au cours de l'imagerie, les algorithmes de recalage utilisent, quel que soit le patient, une matrice de passage initiale unique déterminée une fois pour toutes à l'aide dudit dispositif

Selon un mode de réalisation, les algorithmes de recalage utilisent un ensemble de matrices de passage initiales limité à l'espace des translations.

Selon un mode de réalisation, les algorithmes de recalage utilisent une matrice de passage initiale et, dans le cas où ledit dispositif permet de déterminer les trois paramètres de rotation de la matrice de passage initiale, les trois paramètres de translation de cette matrice sont définis en choisissant un point arbitraire sur la surface du patient définie sur l'image tridimensionnelle, en affichant ce point selon des vues en coupe et en projection, et en demandant à un opérateur de désigner ce point dans le référentiel du dispositif à l'aide d'un palpeur.

Selon un mode de réalisation, les algorithmes de recalage utilisent une matrice de passage initiale et dans le cas où ledit dispositif permet de déterminer certains paramètres de la matrice de passage initiale, les autres paramètres sont définis en effectuant un recalage fin à partir des positions admissibles ainsi calculées et en ne retenant que la meilleure relation.

L'invention prévoit aussi un dispositif de fixation pouvant s'appliquer à une large série de patients et porter un moyen de repérage ; ce dispositif comporte au moins trois appuis destinés à reposer sur une structure anatomique du patient de sorte qu'une relation prédéterminée de position entre le dispositif et la structure anatomique puisse être établie.

Selon un mode de réalisation, le dispositif comprend :
- un élément comportant un premier appui en forme de V destiné à reposer sur le nasion et deux autres appuis destinés à reposer sur le front au-dessus des sourcils ;
- des premier et deuxième moules épousant la forme des conques des oreilles du patient, destinés à être placés respectivement dans l'une et l'autre des oreilles du patient ;
- une barre de maintien pour maintenir ledit élément dans une position stable, ladite barre de maintien comportant une partie centrale destinée à reposer sur l'élément et deux branches destinées à être couplées auxdits premier et deuxième moules.

Selon un mode de réalisation, le dispositif comporte en outre des bagues coulissantes présentant des tiges pour coupler lesdites branches auxdits premier et deuxième moules.

Selon un mode de réalisation, ledit élément présente un système de crans à plusieurs positions situé entre le premier appui et les deux autres appuis, le système de crans étant destiné à recevoir la partie centrale de la barre de maintien et à permettre le réglage de la pression de maintien du dispositif.

Selon un mode de réalisation, le moyen de repérage comporte deux plaques latérales obliques l'une par rapport à l'autre présentant des éléments de référence.

Selon un mode de réalisation, le moyen de repérage comporte en outre une plaque triangulaire frontale présentant au moins trois trous et/ou au moins un ergot de calibrage.

Selon un mode de réalisation, le dispositif, applicable à une vertèbre lombaire, comprend deux appuis destinés à être placé sur le bord postérieur de l'apophyse épineuse et une pince à mors perpendiculaire à la droite passant par lesdits deux appuis.

Selon un mode de réalisation, le dispositif, applicable à une vertèbre thoracique, comprend deux appuis destinés à être placé sur le bord postérieur de l'apophyse épineuse et une pince à mors faisant, par rapport à la droite passant par les deux appuis, un angle prédéterminé, défini par le niveau de la vertèbre.

L'invention prévoit aussi une pointe de référence, utilisable dans le procédé de l'invention ou en coopération avec le dispositif de l'invention ; la pointe de référence comporte :
une pointe cylindrique présentant un certain diamètre D, destinée à s'insérer dans un trou percé dans un os et présentant sensiblement le même diamètre D,
une collerette portée par la pointe cylindrique, et
un moyen de repérage permettant le repérage d'un point de référence de la pointe de référence.

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
la figure 1 est une vue en perspective d'un élément d'un dispositif selon un premier mode de réalisation de l'invention ;
la figure 2 est une vue de dessus d'un autre élément du dispositif selon le premier mode de réalisation de l'invention ;
la figure 3 illustre une variante de l'élément de la figure 2, ainsi que d'autres éléments du dispositif selon le premier mode de réalisation de la présente invention ;
la figure 4 est une représentation du dispositif selon le premier mode de réalisation de l'invention appliqué à un patient ;
les figures 5A et 5B représentent un dispositif selon le premier mode de réalisation de l'invention pourvu d'un moyen de repérage particulier ;
les figures 6A et 6B représentent deux vues en perspective d'un dispositif selon un deuxième mode de réalisation de l'invention ;
les figures 7A et 7B représentent un dispositif selon un autre mode de réalisation de l'invention ; et
la figure 8 représente une pointe de référence en position utilisable dans la présente invention.

Divers modes de réalisation de l'invention vont être maintenant décrits.

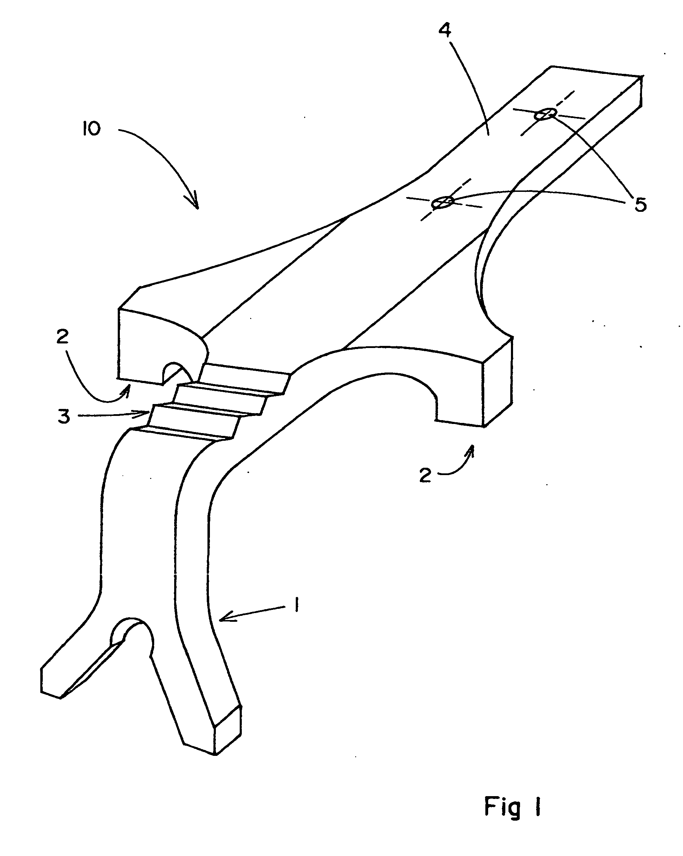

Les figures 1 à 5 illustrent un premier mode de réalisation d'un dispositif selon l'invention, utilisable par exemple en chirurgie ORL, neurochirurgie, radiothérapie du cerveau et en chirurgie maxillo-faciale.

La figure 1 représente un élément 10 de ce dispositif. L'élément 10 de la figure 1 est de forme allongée et présente sur une extrémité un premier appui 1 destiné à reposer sur le nasion. Cet appui a la forme d'un V et présente, comme sur des lunettes, deux surfaces latérales obliques assurant un bon positionnement au niveau de la racine du nez. L'élément 10 présente ensuite une courbure et comporte deux autres appuis 2. Les appuis 2 sont constitués de surfaces légèrement arrondies et sont destinés à s'appuyer sur le front au-dessus des sourcils. En outre, l'élément de la figure 1 présente, entre le premier appui 1 et les deux appuis frontaux 2, un système de crans 3 à plusieurs positions (trois dans l'exemple illustré), dont la fonction sera décrite ci-après. L'élément de la figure 1 se termine, dans l'exemple représenté, par une partie rectiligne 4 qui comporte deux trous de fixation 5. Ces trous sont destinés à fixer par un moyen de fixation quelconque, comme des vis, un moyen de repérage (non représenté en figure 1), comme par exemple un corps de référence porteur de diodes électroluminescentes, ce corps de référence permettant le repérage de la position du dispositif par un système de localisation associé à l'enceinte opératoire. L'homme du métier notera que l'élément 10 de la figure 1 pourrait comporter de manière intrinsèque le moyen permettant de le repérer dans l'enceinte opératoire.

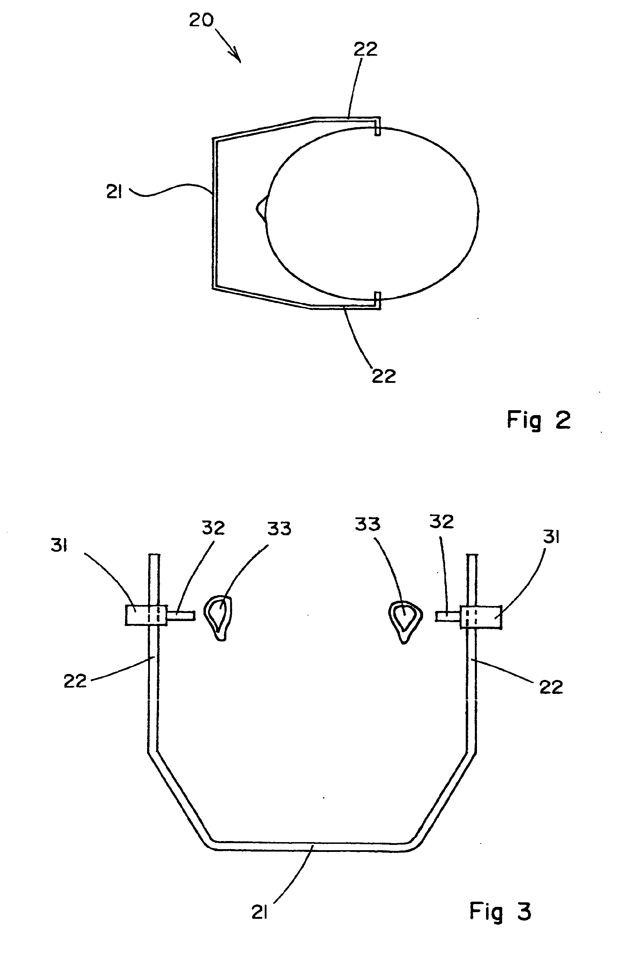

La figure 2 illustre une barre de maintien 20 destinée à coopérer avec l'élément 10 pour le maintenir dans une position stable sur le patient. La barre de maintien 20 est grossièrement en forme de U et présente une partie centrale 21 et deux branches 22. La figure 2 représente la barre de maintien 20 appliquée à la tête d'un patient et l'on voit que, dans ce mode de réalisation, les extrémités des branches 22 sont recourbées et s'insèrent dans les oreilles du patient.

La figure 3 représente une variante de réalisation de la barre de maintien 20 dans laquelle les branches 22 reçoivent des bagues 31 se terminant par des tiges 32. Les bagues 31 peuvent coulisser sur les branches 22 de la barre de maintien afin de permettre un réglage adapté à la morphologie du patient. Lorsque la position des bagues est réglée, les bagues sont serrées sur les branches 22 par un moyen de serrage (non représenté) et les tiges 32 des bagues sont insérées dans les oreilles du patient.

L'insertion des extrémités recourbées des branches 22 de la figure 2 ou des tiges 32 de la figure 3 ne s'effectue pas directement dans les oreilles du patient, pour éviter de le blesser. On réalise, par exemple en résine, des moules 33 épousant la forme des conques des oreilles du patient. Ces moules sont placés dans les oreilles du patient et les extrémités des branches 22 ou les tiges 32 des bagues 31 viennent s'insérer dans ces moules. Les moules 33 peuvent être réalisés facilement pour chaque patient mais, en pratique, trois ou quatre types de moules suffisent pour une large population, d'où une économie de temps et de matériel.

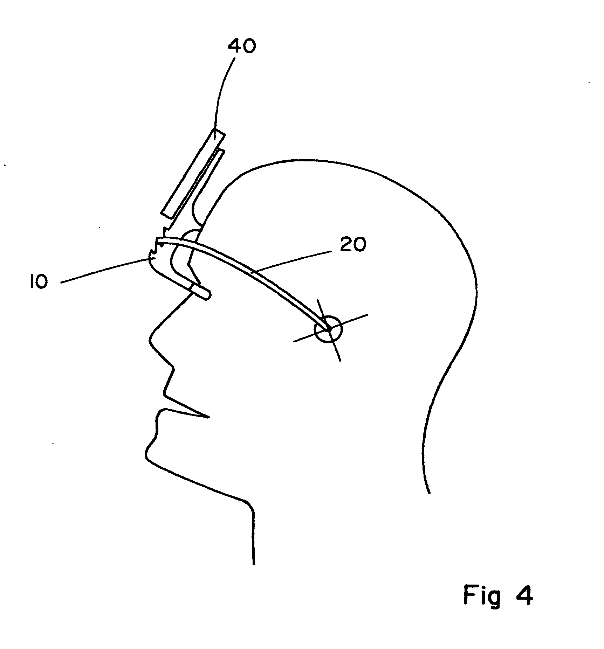

La figure 4 illustre un dispositif selon le premier mode de réalisation de l'invention en position sur un patient. On reconnaît l'élément 10 avec ses appuis anatomiques, la barre de maintien 20 avec ses extrémités couplées aux oreilles du patient. La partie centrale de la barre de maintien 20 s'appuie sur l'un des crans, ici le cran médian, du système de crans à plusieurs positions présenté par l'élément 10 et permet de maintenir l'élément 10 en position tout en réglant la pression de maintien. Ainsi, l'élément 10 est immobilisé, sans qu'il soit nécessaire d'exercer des forces de serrage excessives. En outre, sur cette figure, est représenté un moyen de repérage 40 fixé à l'élément 10 permettant le repérage du dispositif dans l'enceinte opératoire. Enfin, on notera que le dispositif est suffisamment fin et évidé pour ne pas gêner l'action du chirurgien pendant l'opération.

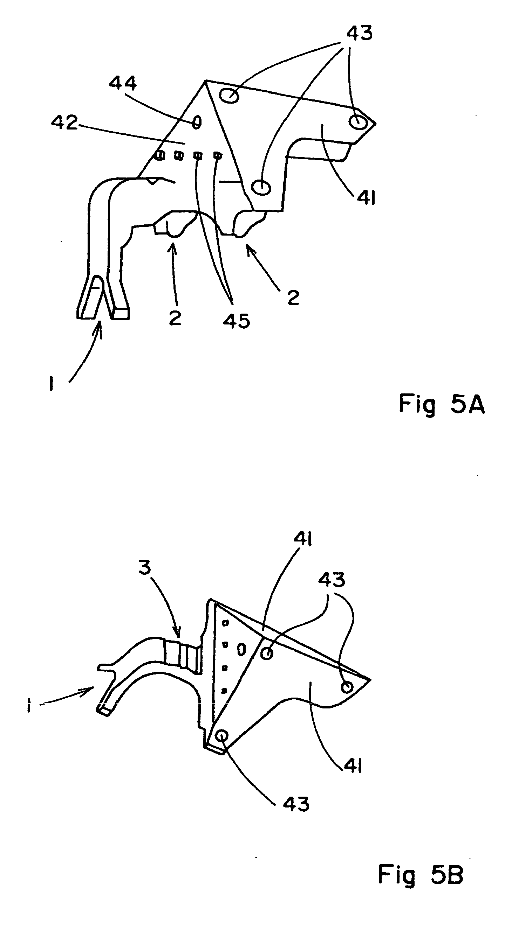

Les figures 5A et 5B représentent deux vues en perspective d'un dispositif selon le premier mode de réalisation de l'invention pourvu d'un moyen de repérage particulier. On y reconnaît l'élément 10 avec ses appuis 1, 2 ; le système de crans 3 de l'élément 10 est également représenté. Ici, le moyen de repérage porté par l'élément 10 comprend un support (non représenté) sur lequel sont fixées par un moyen quelconque, par exemple magnétique, deux plaques latérales 41 obliques l'une part rapport à l'autre. Les plaques latérales 41 présentent des éléments de repérage 43 comme des marqueurs rétro-réfléchissants, repérables par le système de localisation. Dans le mode de réalisation représenté, les plaques sont munies de trois trous circulaires et recouvrent une bande réfléchissante prédécoupée, de telle sorte que la plaque agit comme un masque ne laissant passer que le motif des trous. Un avantage d'un tel moyen de repérage est que, quelle que soit la position du patient, au moins une des plaques obliques 41 est toujours visible et facilement détectée par le système de localisation. On peut donc déplacer de façon importante la tête du patient, sans craindre que le système de localisation perde le suivi du moyen de repérage. En outre, ce moyen de repérage comprend une plaque triangulaire frontale 42 qui présente de façon avantageuse un ou plusieurs trous 43 et un ou plusieurs ergots 44 permettant le calibrage d'outils.

Le dispositif décrit jusqu'à présent s'applique à la chirurgie de la tête, ORL en particulier. L'invention ne se limite pas à ce domaine. De façon générale, la présente invention vise des dispositifs applicables à diverses parties du corps humain, retrouvant une même position avant et après démontage, cette position présentant une relation particulière déterminée par rapport aux structures anatomiques voisines.

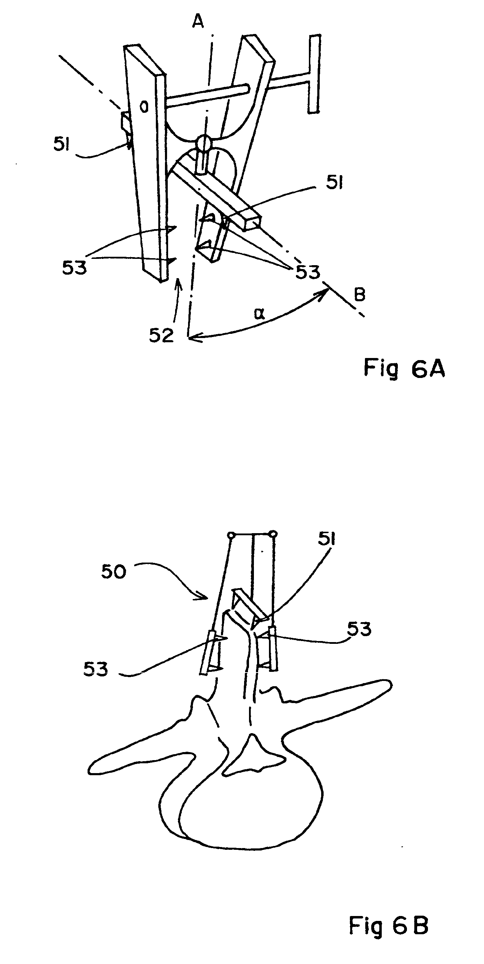

Les figures 6A et 6B illustrent, à titre d'exemple, un autre mode de réalisation de l'invention. Dans ce mode de réalisation, un dispositif selon l'invention s'applique sur une vertèbre. Le dispositif comprend deux appuis 51 destinés à être placés sur le bord postérieur de l'apophyse épineuse. Une pince à mors 52 présente des appuis latéraux 53 classiques et permet de fixer le dispositif sur la vertèbre. L'axe longitudinal A de la pince à mors 50 fait un certain angle α avec une droite B passant par les deux appuis 51. Dans le cas d'une vertèbre lombaire, la pince à mors est perpendiculaire à la droite B et l'angle α est égal à 90°. Dans le cas d'une vertèbre thoracique, la pince à mors fait un angle prédéterminé dépendant du niveau de la vertèbre (par exemple, 30°, 45°, etc.) par rapport à la droite B. Du fait que les appuis 51 reposent sur une partie déterminée et bien définie de la vertèbre, le dispositif selon l'invention présente une relation prédéterminée de position avec la structure anatomique et permet l'application du procédé selon l'invention. La figure 6B illustre le dispositif en position sur une vertèbre.

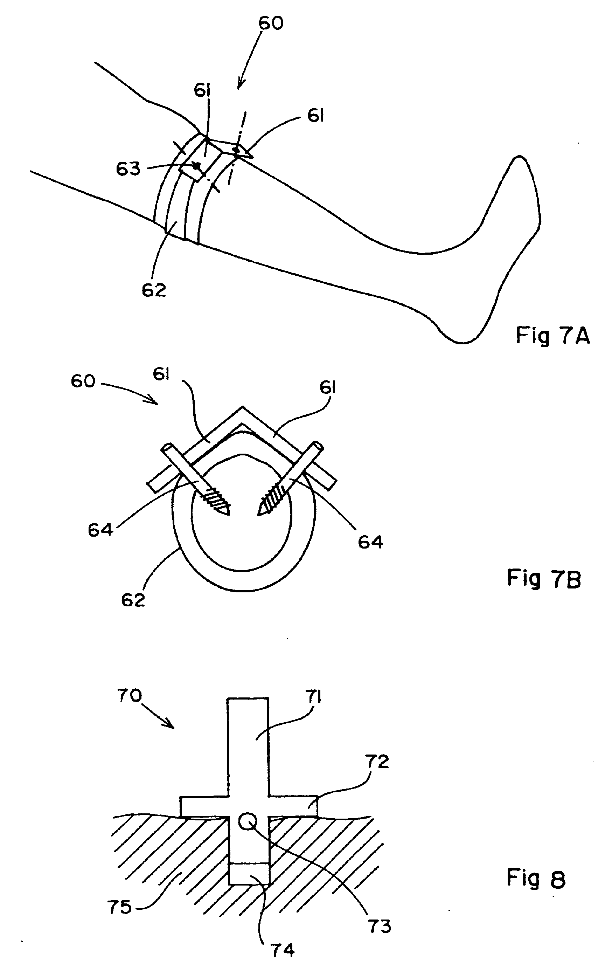

Les figures 7A et 7B représentent un dispositif selon un autre de mode de réalisation. Ici, le dispositif s'applique à un os long, un tibia plus particulièrement. En figure 7A, un dispositif 60 formé par deux parties 61 obliques l'une par rapport à l'autre est placé sur la saillie présentée par la surface externe antérieure du tibia et est serré par une sangle 62. Une ou deux ouvertures 63 permettent d'insérer des tubes aux travers desquels des broches pourront être fixées. La figure 7B représente une coupe du dispositif avec ses broches de fixation 64.

Chacun des dispositifs décrits ci-dessus présente, par rapport à l'état de la technique, des appuis différents et/ou en nombre supérieur, choisis pour augmenter la stabilité et permettre l'établissement d'une relation de position prédéterminée entre le dispositif et une structure anatomique du patient. En outre, les dispositifs de l'invention sont applicables à une large série de patients et présentent, quel que soit le patient, une relation de position prédéterminée avec la structure anatomique du patient, comme cela a été indiqué ci-dessus.

La présente invention prévoit aussi un procédé pour recaler le référentiel d'un dispositif selon l'invention placé sur un patient sur le référentiel d'une image tridimensionnelle préenregistrée de ce patient. Pour cela, on effectue un repérage du dispositif de fixation selon l'invention et on utilise sa relation de position prédéterminée avec la structure anatomique du patient pour déterminer la relation entre le référentiel du dispositif et le référentiel de l'image préenregistrée.

Deux cas se présentent, selon que cette relation de position prédéterminée détermine la position de la structure anatomique du patient de façon complète ou non.

Dans le premier cas, le dispositif présente une position unique par rapport à l'anatomie externe du patient. Par exemple, ce sera le cas pour le dispositif applicable à la tête décrit ci-dessus si l'opérateur respecte les appuis prévus et s'il veille à ce que l'appui nasal vienne en contact avec la région intersourcillière le plus haut possible sur le nez. Cette situation présente les avantages suivants. D'abord, elle permet de placer et de retirer le dispositif à volonté, le dispositif restant dans la même position par rapport au patient. Ensuite, cette position unique peut être prédite dans le référentiel des images à l'aide d'outils de simulation. On connaît alors directement la position du dispositif par rapport aux images lors de l'intervention. Dans ce cas, la relation de position prédéterminée entre la position du dispositif et la position du patient est suffisamment précise pour permettre un recalage approprié. On évite ainsi d'effectuer l'étape préalable des procédés antérieurs qui consistait à déterminer la position d'un corps de référence par rapport au patient, d'où un gain de temps et de précision.

Dans le second cas, la relation prédéterminée de position entre le dispositif et la structure anatomique ne fournit pas complètement la position du dispositif par rapport à la structure anatomique et on ne connaît que certains des paramètres de la position du dispositif ou leurs combinaisons. Par exemple, la pince à mors sur la vertèbre peut glisser en translation le long du bord postérieur de l'apophyse. Le support nasal peut également avoir un mouvement de glissement en translation et de légère rotation le long du nez. Dans ce cas, la détermination directe décrite ci-dessus ne permet d'obtenir qu'un recalage grossier et insuffisant. On doit alors compléter le procédé de recalage par un recalage fin de surfaces, afin de déterminer avec précision la position du dispositif sur la structure anatomique.

Cette détermination, qui équivaut à trouver la matrice de passage entre les deux référentiels, peut être réalisée de plusieurs manières.

Comme cela a été mentionné ci-dessus, des recalages fins de l'état de la technique utilisent de façon classique des algorithmes de recalage itératifs pour trouver la matrice de passage M entre le référentiel associé au patient et le référentiel associé aux images médicales. Ces algorithmes établissent une correspondance entre des points de la surface anatomique du patient déterminés par rapport au système de référence fixé au patient et des points de cette même surface dans l'image préenregistrée. Dans l'art antérieur, on a souvent utilisé comme point de départ des algorithmes une matrice initiale, définie par le repérage de plusieurs points anatomiques dans le système de localisation. Ce repérage de points anatomiques prend du temps, mais, surtout il est imprécis et peut être source d'erreurs importantes. Ainsi, si la matrice initiale est trop éloignée de la matrice cherchée, les algorithmes peuvent converger vers des minima locaux et fournir des solutions erronées, voire aberrantes. Une solution à ce problème serait d'utiliser des algorithmes de recherche exhaustive, pour lesquels la convergence de recalage de surfaces est réalisée à partir, par exemple, de 20⁶ matrices initiales et où le meilleur résultat est retenu. Le temps nécessité par les calculs est très long. Le dispositif selon l'invention fournit une bonne approximation de la position de la surface considérée du patient, et permet d'obtenir une valeur initiale de la matrice de passage M très proche de sa valeur finale. En d'autres termes, le recalage grossier susmentionné sert à fournir une base de départ aux algorithmes de recalage de surfaces. On peut alors utiliser des algorithmes plus simples, qui convergent rapidement de manière fiable.

Pour déterminer la matrice initiale, plusieurs modes sont possibles.

Si, lors de l'examen d'imagerie, le patient est placé dans une position anatomique relativement constante, il est possible de déterminer la matrice M de passage entre les deux référentiels une fois pour toutes sur un patient quelconque et la stocker dans un fichier. Pour tout nouveau patient, ce fichier est lu pour constituer la matrice initiale servant aux algorithmes de recalage, ce qui est simple à mettre en oeuvre et procure des avantages en gain de temps importants.

Si le patient, lors de l'examen d'imagerie, n'est susceptible de présenter qu'un déplacement en translation, on peut procéder de deux façons différentes.

Selon l'art antérieur, pour mettre en correspondance deux surfaces, on utilisera par exemple 20 valeurs initiales selon chacun des 6 degrés de liberté. Ceci correspond à un calcul à 20⁶ (64000000) matrices initiales. Si on utilise des informations initiales grâce au dispositif selon l'invention, pour se limiter par exemple aux déplacements par translation, on n'aura plus que 20³ (8000) matrices initiales à partir desquelles on va réaliser le recalage fin, pour ne retenir que le meilleur résultat.

Dans une autre façon de procéder dans le cas d'un déplacement en translation, on repère automatiquement dans le référentiel des images, à partir d'un modèle de surface construit sur l'image tridimensionnelle, la position d'un point de la surface (par exemple, le point le plus avant ou le plus arrière de la surface, ou un point situé au milieu du nez) ; au début de l'intervention, on affiche à l'écran le point défini de manière informatique sur différentes vues en coupes et projections tridimensionnelles et le chirurgien doit désigner ce même point avec un palpeur repéré par le système de localisation pour suivre la translation du point dans les images.

Egalement, dans un mode avantageux pour déterminer la matrice initiale, on utilise un traitement informatique de l'image préenregistrée pour définir mathématiquement, sur cette image, l'ensemble des positions stables que peut prendre le dispositif de fixation de l'invention sur la surface anatomique du patient. Des méthodes d'optimisation géométrique utilisées pour résoudre les problèmes d'insertion de pièces mécaniques peuvent être utilisées à cet effet. L'ensemble des positions stables se réduit souvent à un nombre restreint de positions (20 par exemple) situées sur une partie de trajectoire ou de surface dont la dimension n'est plus que 1, 2, 3 ou 4 au lieu de 6. A partir de chacune des solutions obtenues en réalisant le recalage fin à partir des positions possibles, on retient la solution qui donne les meilleurs résultats en termes d'erreur résiduelle.

Enfin, on peut parfois souhaiter connaître avec beaucoup de précision la position du dispositif selon l'invention par rapport au patient. On peut aussi, après avoir retiré le dispositif, souhaiter le replacer exactement à la même position que précédemment. Dans ce cas, on peut placer sur le patient, en plus d'un des dispositifs de fixation précédemment décrits, un autre corps de référence, de position entièrement stable par rapport à la structure anatomique du patient et repérer ou vérifier la position du dispositif de l'invention par rapport à ce deuxième corps de référence.

Par exemple, dans le cas de la chirurgie de la tête, on peut utiliser un moulage dentaire du type déjà décrit et repérer la position du dispositif selon l'invention par rapport à ce moulage. Le moulage dentaire est alors considéré comme une référence absolue par rapport au patient car sa position est théoriquement plus stable. Comme le moulage dentaire est encombrant, il peut être placé par exemple au début de l'intervention pour définir la position exacte du dispositif de l'invention, puis retiré lors de l'intervention. Il peut être replacé chaque fois qu'un léger décalage du dispositif selon l'invention est suspecté et utilisé pour une éventuelle correction.

On peut aussi placer, de manière fixe sur la structure anatomique du patient, au moins trois structures ponctuelles et repérer la position de ces points de référence au début de l'intervention. Au cours de l'intervention, lorsque l'utilisateur suspecte un déplacement potentiel du dispositif de fixation de l'invention ou qu'il souhaite déterminer sa position avec une grande précision, la position de ces points de référence est déterminée et un recalage est effectué entre le référentiel du dispositif et les points de référence. Ces structures ponctuelles peuvent être classiquement des marques dessinées sur la peau, ou des points anatomiques particuliers tels que des jonctions dentaires, ou encore des structures matérielles apposées à la structure anatomique.

De façon avantageuse, ces structures ponctuelles peuvent être des petits trous d'un diamètre D de 1 millimètre environ percés dans un os et que l'on vient détecter par le système de localisation avec une pointe de référence de diamètre D identique. La figure 8 illustre une telle pointe de référence. La pointe de référence 70 comporte une partie cylindrique 71 de diamètre D ainsi qu'une collerette 72 portée par la pointe cylindrique. Elle comporte aussi un moyen de repérage (non représenté) permettant de déterminer la position d'un point de référence 73 par le système de localisation. Lors de son utilisation, on insère la pointe de référence dans un trou de même diamètre 74 percé dans un os 75 ; la collerette 72 limite la pénétration de la pointe cylindrique dans l'os et permet de placer avec précision le point de référence 73 sur la surface de l'os. Cet agencement est avantageux et peut être utilisé en coopération avec un des dispositifs précédents ou dans le procédé de l'invention.

Bien que l'invention ait été décrite dans le cadre d'un nombre limité de modes de réalisation, la présente invention n'est pas limitée à ces modes de réalisation particuliers et est susceptible de diverses variantes et modifications qui apparaîtront à l'homme de l'art.

## Revendications

1. Dispositif de fixation pouvant s'appliquer à une large série de patients et porter un moyen de repérage, comportant au moins trois appuis (1, 2, 51, 53) destinés à reposer sur une structure anatomique du patient de sorte qu'une relation prédéterminée de position entre le dispositif et la structure anatomique puisse être établie, **caractérisé en ce qu'**il comprend :
- un élément (10) comportant un premier appui en forme de V (1) destiné à reposer sur le nasion et deux autres appuis (2) destinés à reposer sur le front au-dessus des sourcils ;
- des premier et deuxième moules (33) épousant la forme des conques des oreilles du patient, destinés à être placés respectivement dans l'une et l'autre des oreilles du patient ;
- une barre de maintien (20) pour maintenir ledit élément (10) dans une position stable, ladite barre de maintien comportant une partie centrale (21) destinée à reposer sur l'élément (10) et deux branches (22) destinées à être couplées auxdits premier et deuxième moules.

2. Dispositif selon la revendication 1, comportant en outre des bagues coulissantes (31) présentant des tiges (32) pour coupler lesdites branches (22) auxdits premier et deuxième moules.

3. Dispositif selon la revendication 1, dans lequel l'élément (10) présente un système de crans à plusieurs positions (3) situé entre le premier appui (1) et les deux autres appuis (2), le système de crans étant destiné à recevoir la partie centrale de la barre de maintien et à permettre le réglage de la pression de maintien du dispositif.

4. Dispositif selon la revendication 1, dans lequel le moyen de repérage comporte deux plaques latérales obliques (41) l'une par rapport à l'autre présentant des éléments de référence (43).

5. Dispositif selon la revendication 4, dans lequel le moyen de repérage comporte en outre une plaque triangulaire frontale (42) présentant au moins trois trous (44) et/ou au moins un ergot (45) de calibrage.

6. Procédé pour recaler le référentiel d'un dispositif placé sur un patient au cours d'une intervention avec le référentiel d'une image tridimensionnelle préenregistrée de ce patient, comprenant les étapes suivantes :
a) placer sur le patient un dispositif de fixation pouvant s'appliquer à une large série de patients et porter un moyen de repérage, destiné à reposer sur une structure anatomique du patient de sorte qu'une relation prédéterminée de position entre le dispositif et la structure anatomique puisse être établie :
b) repérer la position dudit dispositif par rapport à un système de localisation ; et
c) à l'aide du repérage dudit dispositif et de la relation de position prédéterminée du dispositif avec la structure anatomique du patient, déterminer une relation entre le référentiel du dispositif et le référentiel de ladite image,
**caractérisé en ce que** l'étape consistant à déterminer la relation entre le référentiel du dispositif et le référentiel de ladite image comprend les étapes suivantes :
déterminer, par rapport audit dispositif, la position de points appartenant à la surface anatomique du patient,
utiliser des algorithmes de recalage itératifs pour déterminer dans l'image préenregistrée la position de cette surface anatomique, ces algorithmes utilisant la relation de position prédéterminée du dispositif avec la structure anatomique pour débuter leur recherche, et
déterminer une matrice de passage définissant la relation entre le référentiel du dispositif et le référentiel de ladite image.

7. Procédé selon la revendication 6, dans lequel, dans le cas où le patient garde une position sensiblement stable au cours de l'imagerie, les algorithmes de recalage utilisent, quel que soit le patient, une matrice de passage initiale unique déterminée une fois pour toutes à l'aide dudit dispositif.

8. Procédé selon la revendication 6, dans lequel les algorithmes de recalage utilisent un ensemble de matrices de passage initiales limité à l'espace des translations.

9. Procédé selon la revendication 6, dans lequel les algorithmes de recalage utilisent une matrice de passage initiale et dans lequel, dans le cas où ledit dispositif permet de déterminer les trois paramètres de rotation de la matrice de passage initiale, les trois paramètres de translation de cette matrice sont définis en choisissant un point arbitraire sur la surface du patient définie sur l'image tridimensionnelle, en affichant ce point selon des vues en coupe et en projection, et en demandant à un opérateur de désigner ce point dans le référentiel du dispositif à l'aide d'un palpeur.

10. Procédé selon la revendication 6, dans lequel les algorithmes de recalage utilisent une matrice de passage initiale et dans lequel, dans le cas où ledit dispositif permet de déterminer certains paramètres de la matrice de passage initiale, les autres paramètres sont définis en effectuant un recalage fin à partir des positions admissibles ainsi calculées et en ne retenant que la meilleure relation.

11. Utilisation du dispositif selon la revendication 1 ou mise en oeuvre du procédé selon la revendication 6, en coopération avec une pointe de référence (70) comportant :
une pointe cylindrique (71) présentant un certain diamètre D, destinée à s'insérer dans un trou (74) percé dans un os (75) et présentant sensiblement le même diamètre D,
une collerette (72) portée par la pointe cylindrique (71), et
un moyen de repérage permettant le repérage d'un point de référence (73) de la pointe de référence (70).

## Claims

1. A fastening device that can apply to a wide series of patients and support a marking means, including at least three bearings (1, 2, 51, 53) intended for resting upon an anatomic structure of the patient so that a predetermined position relation between the device and the anatomic structure can be established, **characterized in that** it includes:
- an element (10) including a first V-shaped bearing (1) intended for resting upon the nasion and two other bearings (2) intended for resting upon the forehead above the eyebrows;
- first and second moulds (33) following the shape of the patient's ear conchas, intended for being respectively placed in one or the other of the patient's ears;
- a holding bar (20) for holding said element (10) in a stable position, said holding bar including a central portion (21) intended for resting upon the element (10) and two branches (22) intended for being coupled to said first and second moulds.

2. The device of claim 1, further including sliding rings (31) having rods (32) for coupling said branches (22) to said first and second moulds.

3. The device of claim 1, wherein said element (10) has a multiple-position notch system (3) located between the first bearing (1) and the two other bearings (2), the notch system being intended for receiving the central portion of the holding bar and for enabling setting of the device hold pressure.

4. The device of claim 1, wherein the marking means includes two lateral plates (41) oblique with respect to each other exhibiting reference elements (43).

5. The device of claim 4, wherein the marking means further includes a front triangular plate (42) having at least three holes (44) and/or at least one calibration pin (45).

6. A method for readjusting the reference frame of a device placed on a patient during an intervention with the reference frame of a pre-recorded three-dimensional image of this patient, including the steps of:
a) placing on the patient a fastening device that can apply to a wide series of patients and support a marking means, intended for resting upon an anatomic structure of the patient so that a predetermined position relation between the device and the anatomic structure can be established,;
b) finding the position of said device with respect to a localization system; and
c) by means of the localization of said device and of the predetermined position relation of the device with the patient's anatomic structure, determining a relation between the reference frame of the device and the reference frame of said image,
**characterized in that** the step of determining the relation between the reference frame of the device and the reference frame of said image includes the steps of:
determining, with respect to said device, the position of points belonging to the patient's anatomic surface,
using iterative readjustment algorithms to determine in the pre-recorded image the position of this anatomic surface, these algorithms using the predetermined position relation of the device with the anatomic structure to begin their search, and
determining a transfer array defining the relation between the reference frame of the device and the reference frame of said image.

7. The method of claim 6, wherein, in the case where the patient keeps a substantially stable position during the imaging, the readjustment algorithms use, whoever the patient, a single initial transfer array determined once and for all by means of said device.

8. The method of claim 6, wherein the readjustment algorithms use a set of initial transfer arrays limited to the space of translations.

9. The method of claim 6, wherein the readjustment algorithms use an initial transfer array and, in the case where said device enables determining the three rotation parameters of the initial transfer array, the three rotation parameters of the array are defined by choosing an arbitrary point on the patient's surface defined on the three-dimensional image, displaying this point according to cross-section and projection views, and asking an operator to designate this point in the reference frame of the device by means of a sensor.

10. The method of claim 6, wherein the readjustment algorithms use an initial transfer array and, in the case where said device enables determining certain parameters of the initial transfer array, the other parameters are defined by performing a fine readjustment based on the admissible positions so calculated and only keeping the best relation.

11. Use of the device of claim 1 or implementation of the method of claim 6 in cooperation with a reference pin (70), including:
a cylindrical tip (71) having a given diameter D, intended for inserting into a hole (74) drilled in a bone (75) and substantially having the same diameter D,
a flange (72) supported by the cylindrical tip (71), and
a marking means enabling localization of a reference point (73) of the reference pin (70).

## Patentansprüche

1. Befestigungsvorrichtung, die bei einer großen Zahl von Patienten verwendbar ist und ein Markiermittel tragen kann, mit wenigstens drei Auflage- bzw. Stützorganen (1,2, 51, 53) zum Aufruhen auf einer anatomischen Struktur des Patienten, derart dass eine vorbestimmte Lagebeziehung zwischen der Vorrichtung und der anatomischen Struktur aufgestellt werden kann, **dadurch gekennzeichnet dass** die Vorrichtung umfasst:
- ein Element (10), welches ein erstes V-förmiges Auflage- bzw, Stützorgan (1) zum Aufruhen auf der Nase und zwei andere zum Aufliegen auf der Stirn über den Augenbrauen bestimmte Auflage- bzw Stützorgane (2) umfasst;
- erste und zweite Formkörper (33) von den Ohrmuscheln des Patienten angepasster Form, zur Anbringung in dem einen bzw. dem anderen Ohr des Patienten ;
- eine Haltestange (20) zur Halterung des Elements (10) in einer stabilen Lage, wobei die Haltestange ein zum Aufruhen auf dem Element (10) bestimmtes Mittelteil (21) und zwei zur Kupplung mit dem ersten und dem zweiten Formkörper bestimmte Zweige bzw Schenkel (22) aufweist

2. Vorrichtung nach Anspruch 1, welche des weiteren Gleitringe (31) mit Stiften bzw Bolzen (32) zur Kupplung der beiden Schenkel (22) mit dem ersten und zweiten Formkörper aufweist,

3. Vorrichtung nach Anspruch 1, bei welcher das Element (10) ein zwischen dem ersten (1) und den beiden anderen Auflage- bzw. Stützorgan(en) (2) gelegenes System von Nuten bzw Kerben mit mehreren Stellungen (3) aufweist, das zur Aufnahme des Mittelteils der Haltestange bestimmt ist und eine Regelung der Halterungsanpressung der Vorrichtung gestattet.

4. Vorrichtung nach Anspruch 1, bei welcher das Markierungsmittel zwei relativ zueinander schräge Seitenplatten (41) aufweist, welche Bezugselemente (43) tragen.

5. Vorrichtung nach Anspruch 4, bei welcher das Markierungsmittel des weiteren eine dreieckige Frontplatte (42) umfasst, welche wenigstens drei Löcher bzw. Öffnungen (44) und/oder wenigstens einen Kalibriernocken bzw -vorsprung (45) aufweist.

6. Verfahren zum Justieren bzw. Rejustieren des Bezugssystems bzw rahmens einer auf dem Patienten im Verlauf einer Intervention angebrachten Vorrichtung, mit dem Bezugssystem bzw, -rahmen eines vor-aufgezeichneten dreidimensionellen Bildes dieses Patienten, das Verfahren umfassend die folgenden Schritte bzw Stufen
a) Anbringen einer Befestigungsvorrichtung auf dem Patienten, die bei einer großen Zahl von Patienten verwendbar ist und ein Markiermittel tragen kann und die zum Aufruhen auf einer anatomischen Struktur des Patienten bestimmt ist, derart dass eine vorbestimmte Lagebeziehung zwischen der Vorrichtung und der anatomischen Struktur aufgestellt werden kann:
b) Auffinden bzw. Lokalisieren der Lage der Vorrichtung relativ bezüglich einem Lokalisierungssystem;
c) Bestimmen einer Beziehung zwischen dem Bezugssystem bzw.-rahmen der Vorrichtung und dem Bezugssystem bzw. -rahmen des genannten Bildes, unter Zuhilfenahme der Lokalisierung der Vorrichtung und der vorbestimmten Lagebeziehung der Vorrichtung mit der anatomischen Struktur des Patienten;
**dadurch gekennzeichnet dass** die aus der Bestimmung der Beziehung zwischen dem Bezugssystem der Vorrichtung und dem Bezugssystem des genannten Bildes bestehende Stufe die folgenden Stufen bzw. Schritte umfasst:
Bestimmen der Lage von zu der anatomischen Oberfläche des Patienten gehörigen Punkten relativ bezüglich der Vorrichtung;
Anwendung iterativer Justier-Algorithmen zur Bestimmung der Lage dieser anatomischen Oberfläche in dem vor-aufgezeichneten Bild, wobei diese Algorithmen die vorbestimmte Lagebeziehung der Vorrichtung mit der anatomischen Struktur für den Beginn ihres Suchverfahrens benutzen; sowie
Bestimmen einer die Beziehung zwischen dem Bezugssystem der Vorrichtung und dem Bezugssystem des Bildes definierenden Überführungsmatrix.

7. Verfahren nach Anspruch 6, bei welchem für den Fall, dass der Patient eine im wesentlichen stabile Lage im Verlauf der Bildaufnahme beibehält, die Justier-Algorithmen unabhängig vom jeweiligen Patienten eine einzige, einmal für alle Patienten mithilfe der genannten Vorrichtung bestimmte Anfangs-Überführungsmatrix anwenden.

8. Verfahren nach Anspruch 6, bei welchem die Justier-Algorithmen eine auf den Raum den Translationen begrenzte Gruppe anfänglicher Überführungsmatrizen anwenden.

9. Verfahren nach Anspruch 6, bei welchem die Justier-Algorithmen eine Anfangs-Überführungsmatrix anwenden, und bei welchem für den Fall, dass die Vorrichtung die Bestimmung der drei Rotations-Parameter der anfänglichen Überführungsmatrix gestattet, die drei Translationsparameter dieser Matrix definiert werden, indem man einen willkürlichen Punkt auf der in dem dreidimensionalen Bild definierten Oberfläche des Patienten wählt, diesen Punkt gemäß Schnitt- und Projektionsansichten anzeigt, und einen Operator auffordert, diesen Punkt in dem Bezugssystem der Vorrichtung mithilfe eines Messfühlers zu bezeichnen.

10. Verfahren nach Anspruch 6, bei welchem die Justier-Algorithmen eine anfängliche Überführungsmatrix anwenden und bei welchem für den Fall, dass die Vorrichtung die Bestimmung gewisser Parameter der anfänglichen Überführungsmatrix gestattet, die anderen Parameter definiert werden durch Vornahme einer Fein-Justierung auf der Grundlage der so berechneten zulässigen Lagen, und hieraus nur die beste Beziehung beibehalten wird

11. Verwendung der Vorrichtung nach Anspruch 1 oder Ausführung des Verfahrens nach Anspruch 6, im Zusammenwirken mit einer Bezugs-oder Referenzspitze (70), welche umfasst:
eine zylindrische Spitze (71) mit einem bestimmten Durchmesser D, welche zum Einführen in ein Loch (74) bestimmt ist, das in einen Knochen (75) gebohrt ist und im wesentlichen denselben Durchmesser D aufweist;
einen von der zylindrischen Spitze (71) getragenen Bund bzw. Flansch (72), sowie
ein Markiermittel, welches die Markierung bzw Lokalisierung eines Bezugs- bzw Referenzpunkts (73) der Bezugs- bzw Referenzspitze (70) gestattet
